Europäisches Patentamt

European Patent Office

Office européen des brevets

(11) Veröffentlichungsnummer: **0 320 623**
**A1**

(12) ## EUROPÄISCHE PATENTANMELDUNG

(21) Anmeldenummer: 88118782.7

(51) Int. Cl.4: **A61B 5/06 , G01V 3/10**

(22) Anmeldetag: 11.11.88

(30) Priorität: 14.12.87 DE 3742298

(43) Veröffentlichungstag der Anmeldung:
21.06.89 Patentblatt 89/25

(84) Benannte Vertragsstaaten:
AT BE CH DE FR GB LI LU NL

(71) Anmelder: **Pulsotronic Merten GmbH & Co. KG**
**Kaiserstrasse 150 Postfach 100663**
**D-5270 Gummersbach(DE)**

(72) Erfinder: **Chwieralski, Alfred, Dr.**
**Frankfurter Strasse 646**
**D-5000 Köln 91(DE)**
Erfinder: **Dondorf, Wolfgang, Dipl.-Ing.**
**Listringhauser Strasse 18**
**D-5277 Marienheide-Dannenberg(DE)**
Erfinder: **Jacob, Lothar, Dipl.-Ing.**
**Nürsche 17**
**D-5222 Morsbach(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Vorrichtung zur Lage-Ortung eines Katheters oder einer Sonde in einem Organ eines Lebewesens.**

(57) Vorrichtung zur Lage-Ortung eines Katheters (12) oder einer Sonde in einem Körperorgan, z.B. Gefäß-Katheter, Dünndarmsonde oder dgl..

Zur röntgenstrahlenfreien sowie ortsunabhängigen Katheter- bzw. Sondenkontrolle wird eine Vorrichtung vorgeschlagen, die aus einer metallischen Katheter-bzw. Sondenmarkierung (14,15,16,17) mit einer Permeabilität > > 1 sowie einem, ein elektromagnetisches Feld erzeugenden Metalldetektor (11) besteht.

Die erfindungsgemäße Vorrichtung arbeitet in der Weise, daß das elektromagnetische Feld des Metalldetektors (11) in den Körper eindringt und durch die Markierung (14,15,16,17) beeinflußt wird. Die Veränderung der Suchspulenparameter wird optisch und/oder akustisch ausgewertet.

Die Vorrichtung ist handlich, einfach in der Anwendung und ungefährlich.

Fig.1

## Vorrichtung zur Lage-Ortung eines Katheters oder einer Sonde in einem Organ eines Lebewesens

Die Erfindung bezieht sich auf eine Vorrichtung zur Lage-Ortung eines Katheters oder einer Sonde in einem Körperorgan, z.B. Gefäßkatheter, Dünndarmsonde oder dergleichen.

Die bisher übliche Methode zum Orten eines Katheters oder einer Sonde im Körper ist die Röntgenkontrolle, beispielsweise durch eine Thorax-Übersichtsaufnahme. Für diesen Zweck sind Katheter oder Sonden röntgendicht ausgebildet. Auf diese Weise können z.B. bei der Katheterisierung der Vena cava mögliche Komplikationen, besonders in der Schulter-Halsregion, erkannt und vermieden werden. Nachteilig bei der Röntgenmethode ist die Strahlenbelastung und der apparative Aufwand. Deshalb soll auch im klinischen Einsatz z.B. bei Schwangeren von dieser Methode möglichst kein Gebrauch gemacht werden. Ferner steht in akuten Fällen nicht immer eine Röntgenanlage zur Verfügung.

Diesen gebotenen Anwendungsbeschränkungen steht jedoch der Wunsch gegenüber, eine uneingeschränkte Katheter- bzw. Sonden -Ortung durchführen zu können.

Im außerklinischen Einsatz muß bei Notfallpatienten in der Regel auf eine Lagekontrolle des Katheters ganz verzichtet werden, um sofort hochwirksame Medikamente mit gutem Verdünnungseffekt injizieren zu können. Daraus ergibt sich ein relativ hoher Prozentanteil an cranialen bzw. candialen Katheter-Fehllagen. Derartige, durch Katheter-Fehllagen erfolgte Fehlinjektionen können zu Venenverödungen und schwerwiegenden Organschädigungen führen. Bei konzentrierten Lösungen besteht die Gefahr von Venenwandreizungen und Thrombosen.

Die Aufgabe der Erfindung besteht darin, eine Vorrichtung zum Orten eines Katheters oder einer Sonde zu schaffen, die mit geringem apparativen Aufwand auskommt, leicht und handlich ist, eine hohe Treffsicherheit aufweist und ohne schädliche Strahlung arbeitet.

Die Aufgabe wird erfindungsgemäß bei einer Vorrichtung der eingangs beschriebenen Art durch die im Kennzeichen des Hauptanspruches aufgeführten Maßnahmen gelöst.

Weiterbildungen der Erfindung sind in den Unteransprüchen beschrieben

Die Erfindung geht von der Überlegung aus, daß ein Metallteile aufweisender Katheter mit Hilfe eines magnetischen Feldes aufgespürt werden kann, wenn einerseits die Ansprechempfindlichkeit des Metalldetektors so groß ist, daß relativ kleine Metallteile auf eine relativ große Entfernung geortet werden können und andererseits die Metallteile eine maximale Feldbeeinflussung herbeiführen.

Diese Überlegung führt dann zu der Erkenntnis, daß die Ansprechempfindlichkeit der Vorrichtung entscheidend von den elektrischen Eigenschaften des an dem Katheter als Markierung angebrachten Metallteils abhängt. Daraus ist dann letzten Endes erfindungsgemäß abgeleitet worden, daß die Ansprechempfindlichkeit umso größer wird, je höher die Permeabilität und die Wirbelstromverluste in der Markierung sind. Im Ergebnis führt das zu einer maximalen Beeinflussung der elektrischen Parameter der Suchspule, z.B. der Güte, deren Änderung mit Hilfe der Auswerteschaltung durch den Indikator angezeigt wird. Selbstverständlich sind noch andere Auswertemethoden denkbar. Beispielsweise kann die Verstimmung einer abgeglichenen Brücke durch die Markierung ausgewertet werden. Ferner kann der Metalldetektor nach dem Impulsverfahren oder nach dem Schwebungsverfahren arbeiten.

Der Metalldetektor kann in seinem prinzipiellen Aufbau den bekannten, für industrielle Anwendungen, z.B. Maschinensteuerungen entwickelten Metallsuch- bzw. induktiven Schaltgeräten entsprechen. Diese bekannten Metallsuch- bzw. induktiven Schaltgeräte sprechen auf elektrisch leitfähiges Material an, wenn es sich um relativ große Gegenstände handelt, bzw. wenn sie sich in einem geringen Abstand von wenigen Millimetern von der Suchspule befinden.

Diese Voraussetzungen sind jedoch für die subkutane Erkennung eines Katheters oder einer Sonde nicht gegeben. Zur Erzielung eines großen Ansprechabstandes besteht erfindungsgemäß die am Katheter oder an der Sonde vorgesehene Markierung aus hochpermeablem Material ($\mu_i$ > 10.000).

Durch die DE-OS 20 27 408 ist beispielsweise ein Metallsuchgerät zum Auffinden von verdeckten metallischen Einschlüssen im Mauer- oder Betonwerk, z.B. Gas- und Wasserrohre, elektrische Leitungen sowie Eisenbewehrungen, bekannt geworden, das ein elektrisches Streufeld erzeugt. Das Metallsuchgerät ist in der Lage, als kleinsten Metallgegenstand eine dreiadrige Kupferleitung 3 x 1,5 mm² in ca. 4 cm Tiefe zu orten.

Durch die DE-PS 24 46 454 ist ein kontakt- und berührungslos wirkendes Schaltgerät bekannt. Dieses induktive Schaltgerät besitzt einen Oszillator, der ein hochfrequentes, elektromagnetisches Wechselfeld abstrahlt. Durch Eindringen einer geeigneten Steuerfahne in das Wechselfeld wird eine Dämpfung der Amplitude des Oszillators hervorgerufen, die zum Schalten eines elektrischen Verbrauchers ausgewertet wird.

Der Vorteil der erfindungsgemäßen Vorrichtung besteht in der einfachen Handhabung und schnellen Verfügbarkeit und schließt deshalb im außerklinischen Einsatz bei fehlender Röntgenanlage eine Behandlungslücke. Wegen der problem- und gefahrlosen Anwendung bildet die Vorrichung ferner eine sinnvolle Ergänzung zur Röntgenmethode im klinischen Einsatz. Es läßt sich beispielsweise bei ambulanter dringlicher Erstversorgung von Notfallpatienten eine Fehllage des Katheters im Bereich der Vena jugularis interna, Vena subclavia, Vena anonyma sowie Vena jugularis externa durch schrittweises Verfolgen des Katheters mit dem Metalldetektor erkennen.

Mit Hilfe der erfindungsgemäßen Vorrichtung kann auf einfache Weise festgestellt werden, ob ein Katheter in der Vena subclavia den Weg in die Vena anonyma oder den falschen Weg in die Vena jugularis externa bzw. Vena jugularis interna nimmt. Durch Absuchen des Oberkörpers mit dem Metalldetektor wird im einfachsten Fall die augenblickliche Lage des vorderen Katheterendes in der Vene durch ein optisches und/oder akustisches Signal angezeigt. Dieser Vorgang kann beliebig oft wiederholt werden, wobei auch jederzeit eine einfache Möglichkeit zur Lagekontrolle des Katheters gegeben ist.

Die erfindungsgemäße Vorrichtung eignet sich nicht nur zum Orten von Kathetern in oberflächennahen Organen, sondern aufgrund der äußerst sensitiven Eigenschaften auch für tiefergelegene Organe. Der Metalldetektor kann über die Katheter- bzw. Sonden-Ortung hinaus auch im unfallchirurgischen Einsatz zum Metallnachweis bei der operativen Materialentfernung verwendet werden, z.B. um festzustellen, ob sämtliche Metallteile wie Schrauben, Klammern oder Drähte nach einer Knochenoperation entfernt wurden.

Da die Markierung aus einem metallischen Werkstoff besteht, ist der Katheter auch röntgendicht.

Wie eingangs bereits erläutert wurde, hängt die Empfindlichkeit der Vorrichtung stark von den magnetischen Eigenschaften der Kathetermarkierung ab.

Die Ansprüche 2 bis 5 und 16 bis 19 geben einige geeignete Werkstoffe an. Die deutlich höhere Empfindlichkeit der Vorrichtung ist leicht zu veranschaulichen, wenn man die magnetische Leitfähigkeit (Permeabilität $\mu_i$) einiger Werkstoffe vergleicht. Bei Kupfer ist dieser Wert < 1 und liegt bei $1 - 10 \times 10^{-6}$, bei Aluminium ist der Wert > 1 und liegt bei $1 + 22 \times 10^{-6}$. Weichmagnetische und ferritische Werkstoffe haben eine Permeabilität > > 1. Sie liegt bei Ferrit zwischen 4.000 bis 10.000, bei amorphen Metallen (Metallglas) bei ca. 100.000 und reicht bei einigen Nickel-Eisen-Legierungen bis 300.000 (Supermaloy 79 % Nickel, 15 % Eisen, 5 % Molybdän und 0,5 % Mangan). Diese Materialien sind als Katheter- bzw. Sonden-Markierung besonders geeignet.

Besondere Bedeutung kommt in diesem Zusammenhang den amorphen Metallen und Nickel-Eisen-Legierungen auch deshalb zu, weil sie sehr gut verarbeitbar sind und in praktisch jede beliebige Form und Dicke gebracht werden können. Sie sind ausgewalzt zu dünnen Blechen, Streifen, Röhrchen und Spiralen sehr biegsam und ferner chemisch inaktiv.

Die Ansprüche 6 bis 12 und Anspruch 23 geben Beispiele für die konstruktive Ausbildung der Markierungen an. Dabei gilt für die Ansprüche 7, 13, 16 bis 19 sowie für die Ansprüche 22 und 24, daß der Katheter bzw. die Sonde auf der gesamten Länge geortet werden kann. Gemäß Anspruch 12 läßt sich die Empfindlichkeit weiter erhöhen.

Durch die Weiterbildung der Erfindung nach Anspruch 14 und 15 werden Verletzungen der Organe durch die Markierungen ausgeschlossen.

Die Weiterbildung der Erfindung nach den Ansprüchen 16 bis 24 bezieht sich auf einen Katheter mit einem Mandrin. Bei einem derartigen Katheter besteht die Möglichkeit, ihn auch dann noch zu orten, wenn der Mandrin bereits entfernt wurde. Im übrigen gelten die eingangs allgemein unter "Katheter" gemachten Erläuterungen zu den Ansprüchen 1 bis 13 auch für einen Katheter mit einem Mandrin.

Die Ansprüche 25 bis 30 beziehen sich auf die Ausbildung des Metalldetektors. Vorteilhaft stehen die Abmessungen des Metalldetektors in einem angemessenen Verhältnis zu der zu untersuchenden Körperoberfläche, um sowohl eine große Zielgenauigkeit als auch handliche Abmessungen zu erreichen. Dazu gehört einerseits, daß eine seitliche Streuung des elektromagnetischen Feldes nach Möglichkeit vermieden wird (Anspruch 30) und andererseits, daß das Gerät netzunabhängig, mit Batterie, arbeitet (Anspruch 25).

Gemäß Anspruch 26 bis 28 kann der Metalldetektor zum Laden der Batterie direkt oder über einen zwischengeschalteten Kleinspannungsadapter an eine Netzsteckdose für 220 Volt Wechselstrom angeschlossen werden. Ferner besteht die Möglichkeit, den Metalldetektor an ein Ladegerät anzuschließen. Als Steckvorrichtung kann eine 12 Volt Auto-Batterie verwendet werden.

Nachfolgend werden mehrere Ausführungen der Erfindung anhand der Zeichnungen näher erläutert. Es zeigen:

Figur 1 einen durch Punktion einer Armvene eingesetzten Gefäß-Katheter mit einem in der Schlüsselbeinregion angesetzten Metalldetektor zum Orten des Katheters beim Durchgang durch die Vena subclavia der rechten Körperhälfte,

Figur 2 die Vorrichtung zum Orten des Katheters, bestehend aus dem Metalldetektor und der Kathetermarkierung,

Figur 3 einen Gefäß-Katheter mit Kunststoffkanüle, Mandrin-Kappe und Markierung,

Figur 4 den Gefäß-Katheter nach Figur 4, ohne Kunststoffkanüle und mit vorgezogenem Mandrin,

Figur 5 das vordere Ende des Katheters mit einer Markierung,

Figur 6 eine sich über die gesamte Länge des Katheters erstreckende gewendelte Markierung,

Figur 7 eine streifenförmige Markierung,

Figur 8 den Mandrin,

Figur 9 das vordere Ende des Mandrins nach Figur 8, mit einer angesetzten Markierung,

Figur 10 ein Blockschaltbild des Metalldetektors.

Die erfindungsgemäße Vorrichtung besteht aus dem Metalldetektor 11 und wenigstens einer, an dem Katheter 12 oder dem Mandrin 13 angebrachten Markierung 14,15,16,17.

In den Figuren der Zeichnungen ist der Katheter 12 mit einem Mandrin 13 ausgerüstet. Der Katheter 12 besteht aus einem langgestreckten flexiblen Schlauch, der am vorderen Ende offen oder geschlossen sein kann.

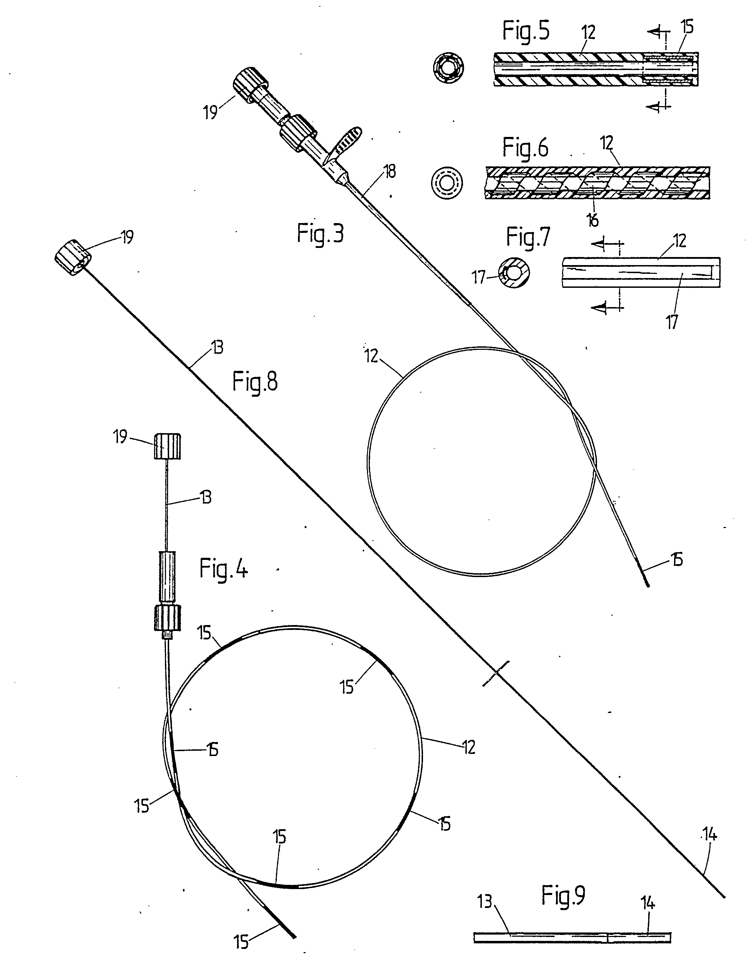

Die in den Figuren 3 und 4 dargestellte Schlinge dient lediglich zur ungekürzten Längendarstellung des Katheters.

In der Praxis ist der Katheter entweder auf einer Trommel aufgewickelt oder befindet sich in seiner gestreckten Länge in einer Peelpackung. In Figur 3 ist der Katheter 12 mit der Einführungskanüle 18 dargestellt. Am hinteren Katheterende befindet sich die Mandrin-Kappe 19. In Figur 4 ist der Mandrin 13 ein Stück aus dem Katheter 12 herausgezogen dargestellt.

Grundsätzlich kann sowohl der Katheter 12 als auch der Mandrin 13 je eine Markierung 14,15,16,17 aufweisen, so daß durch eine dann vorhandene Doppelmarkierung an der gleichen Stelle die Ansprechempfindlichkeit der Vorrichtung vergrößert wird. Es kann jedoch ausreichend sein, wenn entweder am Katheter 12 oder am Mandrin 13 je eine Markierung 14 oder 15,16,17 vorgesehen ist.

Bei einer Teilmarkierung des Katheters 12 bzw. des Mandrins 13 ist die Markierung 14,15 jeweils am vorderen Ende angebracht. Im Gegensatz dazu läuft die Vollmarkierung 16,17 über die gesamte Länge des Katheters 12 bzw. des Mandrins 13 durch. Darunter fällt auch die Lösung, bei der der Mandrin 13 selbst als Markierung ausgebildet ist (siehe Ansprüche 16 bis 19).

Eine weitere Lösung besteht darin, daß jeweils mehrere Markierungen 14,15 auf der Länge des Katheters 12 bzw. des Mandrins 13 verteilt angeordnet sind.

Die Figur 5 zeigt das vordere Ende des Katheters 12 mit einer hülsenartigen Markierung 15. Sie bildet einen Kurzschlußring. Wie erkennbar, ist die Markierung 15 in den Katheter 12 eingebettet. Das kann durch Umspritzen bei der Katheterherstellung erfolgen.

Die Figur 6 zeigt eine wendelartige Markierung 16, die ebenfalls eingebettet ist und sich über die gesamte Katheterlänge erstreckt.

In Figur 7 dient als Markierung 17 ein gerader Blechstreifen, der ebenfalls durch Umspritzen vollständig vom Katheter 12 eingehüllt ist. Bei der Markierung 17 kann es sich um ein Endstück oder um einen durchlaufenden Streifen handeln. Ebenso kann die durchlaufende Markierung 16 als Endstück ' und die Markierung 15 als durchlaufende Hülse oder Röhrchen ausgebildet sein.

Bei einer durchlaufenden Katheter-Markierung 15,16,17 kann u.U. auf die Verwendung eines Mandrins 13 verzichtet werden. Die Markierung 15,16,17 übernimmt dann diese Funktion.

Als Werkstoffe für die Markierungen 14,15,16,17 dienen amorphe Metalle oder NiFe-Legierungen. Sie lassen sich zu dünnen Blechen verarbeiten und durch geeignete Bearbeitungsmethoden zu Streifen, Hülsen, Wendeln oder Drähten, Stiften und Röhrchen weiterverarbeiten.

Die Figur 9 zeigt das vordere Stück eines Stahlmandrins 13, dessen Ende mit der Markierung 14 bestückt ist. Dabei kann es sich um eine aufgesteckte Hülse, um ein Röhrchen oder um einen Stift handeln, der mit dem Stahlmandrin 13 durch Löten oder Schweißen, z B. Stumpfschweißen, verbunden ist. Auf jeden Fall muß sichergestellt sein, daß sich die Markierung 14 nicht vom Mandrin 13 lösen kann.

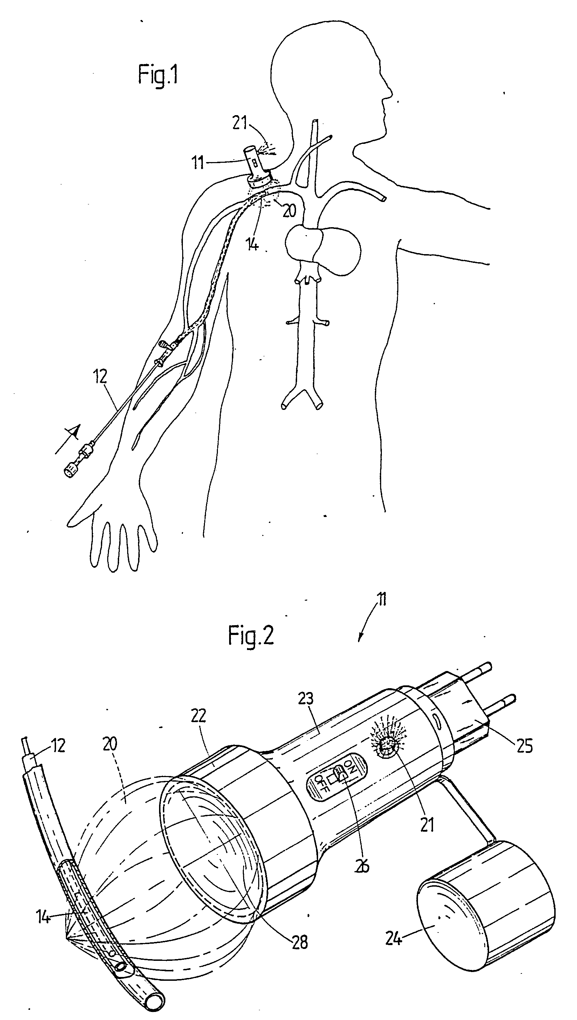

In Figur 1 ist ein Anwendungsbeispiel der Vorrichtung an einem in Umrissen dargestellten menschlichen Körper mit typischem Venenverlauf in der rechten oberen Körperhälfte dargestellt. Eine Armvene ist im Bereich Ellenbeuge punktiert und der Katheter 12 teilweise in die Vene eingeführt. Das vordere Katheterende bzw. dessen Mandrin besitzt eine Markierung 14. Im Bereich des rechten Schlüsselbeins ist auf der Haut über der Vena subclavia der Metalldetektor 11 angelegt. Der Metalldetektor 11 ist eingeschaltet und das austretende elektromagnetische Feld 20 gestrichelt dargestellt. Es dringt in den Körper ein und zeigt durch das Aufleuchten der Signallampe 21 an, daß die Markierung 14 des Katheters 12 sich im Ansprechbereich des Metalldetektors 11 befindet. Durch Abfühlen der Körperoberfläche mit dem Metalldetektor 11 im Bereich des betreffenden Blutgefäßes kann der Weg des Katheters 12 im Körper verfolgt werden. Die Ansprechempfindlichkeit der Vorrichtung sollte ca. 5 cm betragen, so daß auch bei korpulenten Patienten eine sichere Katheterortung möglich ist.

Die Figur 2 zeigt die etwa maßstäbliche, perspektivische Darstellung der Vorrichtung, bestehend aus dem Metalldetektor 11 und der Markierung 14. Die Abmessungen kommen der natürlichen Größe recht nahe. Der Katheter 12 ist zwecks Verdeutlichung vergrößert dargestellt.

Wie aus Figur 2 hervorgeht, sieht der Metalldetektor 11 einer Stableuchte ähnlich Er besitzt einen verbreiterten Kopf 22 und einen schlanken Schaft 23, an dessen Ende eine durch die Schutzkappe 24 abdeckbare Steckvorrichtung 25 und an dessen Schaftumfang die Signallampe 21 und ein Ein- Ausschalter 26 vorgesehen ist.

Der Kopf 22 nimmt die Suchspule 27 auf, die sich in einem einseitig offenen Schalenkern (nicht dargestellt) befindet. Die Anordnung des Schalenkerns ist so gewählt, daß seine offene Seite zur Stirnseite des Kopfes 22 zeigt, welche die aktive Fläche 28 des Metalldetektors 11 bildet, an der das elektromagnetische Feld 20 austreten kann

Der Schaft 23 nimmt die Auswerteschaltung 29, die Schaltung 30 für den Indikator 21,31 sowie die Stromversorgung 32 auf. Bei dem Indikator 21,31 kann es sich, wie bereits beschrieben, um eine Signallampe 21 und/oder um einen akustischen Geber 31 handeln. Die Stromversorgungsschaltung 32 wird durch eine eingebaute wiederaufladbare Batterie gespeist.

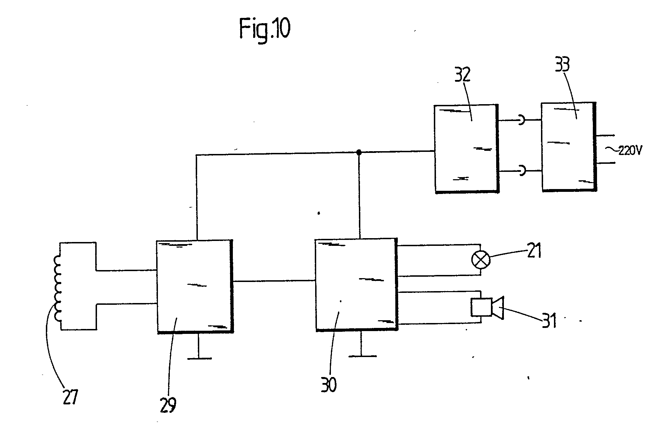

Gemäß Figur 2 läßt sich die Batterie über die Steckvorrichtung 25 an einer Netzsteckdose aufladen. In Figur 10 erfolgt die Aufladung über den Adapter 33.

Die Auswerteschaltung 29 des Metalldetektors 11 besteht aus einem Oszillator, einer Speiseschaltung für den Oszillator, einer Verstärkerstufe, ggf. einer Kippstufe sowie einer Endstufe, die mit der Schaltung 30 für den Indikator 21,31 verbunden ist.

## Ansprüche

1.) Vorrichtung zur Lage-Ortung eines Katheters oder einer Sonde in einem Körperorgan eines Lebewesens, dadurch gekennzeichnet, daß die Vorrichtung wenigstens eine, in der Nähe des vorderen Endes des Katheters (12) vorgesehene metallische Markierung (14,15,16,17) hoher magnetischer Permeabilität > > 1 und einen, eine Suchspule (27), eine Auswerteschaltung (29) und einen Indikator (21,31) enthaltenden Metalldetektor (11) aufweist, der mit Hilfe der Suchspule (27) ein gerichtetes elektromagnetisches Feld erzeugt, auf das der Metalldetektor (11) reagiert, wobei die Markierung (14,15,16,17) das elektromagnetische Feld der Suchspule (27) derart beeinflußt, daß ein Signal erzeugt wird, wenn der Abstand der Markierung von der Suchspule (27) einen Grenzwert unterschreitet.

2.) Vorrichtung zum Orten eines Katheters oder einer Sonde nach Anspruch 1, dadurch gekennzeichnet, daß die Markierung (14,15,16,17) aus ferromagnetischem Material besteht.

3.) Vorrichtung zum Orten eines Katheters oder einer Sonde nach Anspruch 1 oder 2, dadurch gekennzeichnet, daß die Markierung (14,15,16,17) aus amorphem Metall besteht.

4.) Vorrichtung zum Orten eines Katheters oder einer Sonde nach Anspruch 1 oder 2, dadurch gekennzeichnet, daß die Markierung (14,15,16,17) aus weichmagnetischem Material, z.B. einer NiFe- Legierung, besteht.

5.) Vorrichtung zum Orten eines Katheters oder einer Sonde nach Anspruch 1 oder 2, dadurch gekennzeichnet, daß die Markierung (14,15,16,17) aus ferritischem Material besteht.

6.) Vorrichtung zum Orten eines Katheters oder einer Sonde nach einem der Ansprüche 1 bis 5, dadurch gekennzeichnet, daß die Markierung (14,15,16, 17) aus dünnem Blech besteht.

7.) Vorrichtung zum Orten eines Katheters oder einer Sonde nach einem der Ansprüche 1 bis 6, dadurch gekennzeichnet, daß die Markierung (14,15,16, 17) über die gesamte Länge des Katheters (12) angeordnet ist.

8.) Vorrichtung zum Orten eines Katheters oder einer Sonde nach einem der Ansprüche 1 bis 7, dadurch gekennzeichnet, daß die Markierung (14,15,16, 17) aus einem Blechstreifen besteht.

9.) Vorrichtung zum Orten eines Katheters oder einer Sonde nach einem der Ansprüche 1 bis 8, dadurch gekennzeichnet, daß die Markierung (17) aus einem geraden Blechstreifen besteht.

10.) Vorrichtung zum Orten eines Katheters oder einer Sonde nach einem der Ansprüche 1 bis 9, dadurch gekennzeichnet, daß die Markierung (16) wendelartig ausgebildet ist.

11.) Vorrichtung zum Orten eines Katheters oder einer Sonde nach einem der Ansprüche 1 bis 8, dadurch gekennzeichnet, daß die Markierung (15) hülsenartig ausgebildet ist.

12.) Vorrichtung zum Orten eines Katheters oder einer Sonde nach einem der Ansprüche 1 bis 8 sowie 11, dadurch gekennzeichnet, daß die Markierung (15) als Kurzschlußring ausgebildet ist.

13.) Vorrichtung zum Orten eines Katheters oder einer Sonde nach einem der Ansprüche 1 bis 12, dadurch gekennzeichnet, daß mehrere Markierungen (14,15,16,17) über die Länge des Katheters (12) bzw. der Sonde verteilt angeordnet sind.

14.) Vorrichtung zum Orten eines Katheters oder einer Sonde nach einem der Ansprüche 1 bis 13, dadurch gekennzeichnet, daß die Markierung (15,16,17) in den Katheter (12) bzw. der Sonde eingebettet ist.

15.) Vorrichtung zum Orten eines Katheters oder einer Sonde nach einem der Ansprüche 1 bis 14, dadurch gekennzeichnet, daß der Katheter (12) bzw. die Sonde aus zwei ineinandersteckenden Schläuchen besteht, zwischen denen die Markierung (15,16,17) eingebettet ist.

16.) Vorrichtung zum Orten eines Katheters mit einem Mandrin, bei dem der Katheter und/oder der Mandrin eine Markierung aufweist, nach einem der Ansprüche 1 bis 15, dadurch gekennzeichnet, daß der Mandrin (13) aus ferromagnetischem Material besteht.

17.) Vorrichtung zum Orten eines Katheters mit Mandrin nach einem der Ansprüche 1 bis 16, dadurch gekennzeichnet, daß der Mandrin (13) aus amorphem Metall besteht.

18.) Vorrichtung zum Orten eines Katheters nach einem der Ansprüche 1 bis 16, dadurch gekennzeichnet, daß der Mandrin (13) aus weichmagnetischem Material, z.B. einer NiFe-Legierung, besteht.

19.) Vorrichtung zum Orten eines Katheters nach einem der Ansprüche 1 bis 16, dadurch gekennzeichnet, daß der Mandrin (13) aus ferritischem Material besteht.

20.) Vorrichtung zum Orten eines Katheters nach einem der Ansprüche 1 bis 19, dadurch gekennzeichnet, daß das vordere Ende des Mandrins (13) aus ferromagnetischem Material, z.B. amorphem Metall, weichmagnetischem Material wie NiFe-Legierung oder aus ferritischem Material besteht.

21.) Vorrichtung zum Orten eines Katheters nach einem der Ansprüche 1 bis 20, dadurch gekennzeichnet, daß das vordere Ende des Mandrins (13) eine Markierung (14,15,16,17) aus ferromagnetischem Material, z.B. amorphem Metall, weichmagnetischem Material wie NiFe-Legierung oder aus ferritischem Material besteht.

22.) Vorrichtung zum Orten eines Katheters mit einem Mandrin, bei dem der Katheter und/oder der Mandrin eine Markierung aufweist, nach einem der Ansprüche 1 bis 15, dadurch gekennzeichnet, daß die Markierung (14,15, 16,17) über die gesamte Länge des Mandrins (13) angeordnet ist.

23.) Vorrichtung zum Orten eines Katheters mit einem Mandrin nach Anspruch 21 oder 22, dadurch gekennzeichnet, daß der Mandrin (13) einen Kern oder einen Mantel aus ferromagnetischem Material, z.B. amorphem Metall, weichmagnetischem Material wie NiFe-Legierung oder aus ferritischem Material aufweist.

24.) Vorrichtung zum Orten eines Katheters mit einem Mandrin nach Anspruch 22 oder 23, dadurch gekennzeichnet, daß der Mandrin (13) mehrere über die gesamte Länge verteilt angeordnete Markierungen (14,15,16,17) aufweist.

25.) Vorrichtung zum Orten eines Katheters oder einer Sonde nach einem der Ansprüche 1 bis 24, dadurch gekennzeichnet, daß der Metalldetektor (11) als Handgerät mit eingebauter Stromversorgung ausgebildet ist.

26.) Vorrichtung zum Orten eines Katheters oder einer Sonde nach einem der Ansprüche 1 bis 25, dadurch gekennzeichnet, daß der Metalldetektor (11) eine Steckvorrichtung (25) zum Laden der Batterie aufweist.

27.) Vorrichtung zum Orten eines Katheters oder einer Sonde nach Anspruch 26, dadurch gekennzeichnet, daß die Steckvorrichtung (25) als Netzstecker für 220 Volt Wechselstrom ausgebildet ist.

28.) Vorrichtung zum Orten eines Katheters oder einer Sonde nach Anspruch 26, dadurch gekennzeichnet, daß die Steckvorrichtung (25) als Kleinspannungsstecker ausgebildet ist.

29.) Vorrichtung zum Orten eines Katheters oder einer Sonde nach einem der Ansprüche 1 bis 28, dadurch gekennzeichnet, daß der Metalldetektor (11) einen das elektromagnetische Feld erzeugenden Generator bzw. Oszillator aufweist.

30.) Vorrichtung zum Orten eines Katheters oder einer Sonde nach einem der Ansprüche 1 bis 29, dadurch gekennzeichnet, daß die Suchspule (27) innerhalb eines einseitig offenen Schalenkerns angeordnet ist.

Fig.1

Fig.2

Fig.5

Fig.6

Fig.7

Fig.3

Fig.8

Fig.4

Fig.9

Fig.10

~220V

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 416 289 (R.G. BRESLER) * Zusammenfassung; Spalte 1, Zeile 65 - Spalte 2, Zeile 16; Spalte 2, Zeile 38 - Spalte 3, Zeile 4; Spalte 5, Zeilen 8-43; Spalte 11, Zeilen 40-49; Figur 1 * | 1,2,4-6 ,8,12, 25,29, 30 | A 61 B 5/06 G 01 V 3/10 |
| X | US-A-3 847 157 (J.C. CAILLOUETTE) * Insgesamt * | 1,2,4,5 ,7-9 | |
| X | FR-A-2 591 492 (McCORMICK LABORATORIES) * Zusammenfassung * | 1,8,12 | |
| A | EP-A-0 091 577 (S. KUNKE) * Zusammenfassung; Ansprüche 1,21; Figuren 1,2,5-11 * | 1,7,10, 15 | |
| A | US-A-4 526 177 (M.A. RUDY et al.) * Zusammenfassung; Spalte 3, Zeile 49 - Spalte 4, Zeile 46; Figur 1 * | 1,25-30 | |

RECHERCHIERTE SACHGEBIETE (Int. Cl.4)

A 61 B
G 01 V
A 61 M

Der vorliegende Recherchenbericht wurde für alle Patentansprüche erstellt

| Recherchenort | Abschlußdatum der Recherche | Prüfer |
|---|---|---|
| DEN HAAG | 17-03-1989 | FERRIGNO, A. |